Europäisches Patentamt

European Patent Office

Office européen des brevets

(11) Publication number: **0 026 672**
**B1**

(12) **EUROPEAN PATENT SPECIFICATION**

(45) Date of publication of patent specification: 05.06.85

(21) Application number: **80303452.9**

(22) Date of filing: **30.09.80**

(51) Int. Cl.⁴: **C 12 N 11/08, C 12 N 9/38,**
**A 23 C 9/12, A 23 C 21/02**

(54) **Immobilized lactase and its use in hydrolysing lactose.**

(30) Priority: **02.10.79 JP 127754/79**

(43) Date of publication of application:
**08.04.81 Bulletin 81/14**

(45) Publication of the grant of the patent:
**05.06.85 Bulletin 85/23**

(84) Designated Contracting States:
**BE DE FR GB IT NL**

(56) References cited:
**DE-A-2 818 086**
**FR-A-2 198 953**
**FR-A-2 381 059**
**FR-A-2 424 282**
**GB-A-1 557 944**
**JP-A-54 119 084**
**US-A-3 767 531**
**US-A-3 983 000**

(73) Proprietor: **SUMITOMO CHEMICAL COMPANY,**
**LIMITED**
**15 Kitahama 5-chome Higashi-ku**
**Osaka-shi Osaka 541 (JP)**

(72) Inventor: **Hirohara, Hideo**
**2-1-146, Kuwatacho Ibaraki-shi**
**Osaka (JP)**
Inventor: **Yamamoto, Hidefumi**
**26-506, Tamagawa 1-chome Takatsuki-shi**
**Osaka (JP)**
Inventor: **Kawano, Emiko**
**4-14, Noda 6-chome Fukushima-ku**
**Osaka (JP)**
Inventor: **Nabeshima, Shigeyasu**
**2-1-344 Kuwatacho Ibaraki-shi**
**Osaka (JP)**
Inventor: **Mitsuda, Satoshi**
**2-40, Hirata 1-chome Ibaraki-shi**
**Osaka (JP)**
Inventor: **Nagase, Tsuneyuki**
**12-11, Miyanokawahara 5-chome Takatsuki-shi**
**Osaka (JP)**

(74) Representative: **Myerscough, Philip Boyd et al**
**J.A.Kemp & Co. 14, South Square Gray's Inn**
**London, WC1R 5EU (GB)**

Courier Press, Leamington Spa, England.

## Description

The present invention relates to a water-insoluble and enzymatically active immobilized lactase. More particularly, the invention pertains to a stable and water-insoluble immobilized lactase having a high lactose-hydrolyzing activity, which is prepared by immobilizing a lactase derived from *Aspergillus oryzae* on a macroporous amphoteric phenol-formaldehyde type ion-exchange resin.

When an enzyme, which *per se* is a catalyst for a reaction in homogeneous aqueous solution, is immobilized, it becomes possible to be used repeatedly and continuously. In reflection of such usefulness of an immobilized enzyme in an industrial application, there have recently been developed a number of enzyme-immobilization techniques [C. R. Zaborsky: Immobilized Enzymes, C.R.C. Press, (1973); Immobilized Enzymes, edited by Ichiro Chihata, published by Kodansha (1975)].

Hitherto known methods for producing immobilized enzymes may roughly be divided into the following four methods.

    (1) Adsorption method
    (2) Covalent attachment method
    (3) Entrapment method
    (4) Crosslinking method

Since each method has both advantages and disadvantages over the others, it is hard to tell which one is superior to the others. Moreover, there has not yet been provided the enzyme-immobilization carrier and the enzyme-immobilization method which are applicable to any and all diverse enzymes. In practice, a suitable carrier and a method must individually be selected depending upon a particular purpose so that the excellent immobilized enzyme which serves the intended purpose may be obtained.

In addition thereto, attention should be paid to the fact that even the enzymes which are classified in the same category and given an identical enzyme number in accordance with the Report of the Commision on Enzymes of the International Union of Biochemistry, 1964, may be quite different from each other in molecular weight and enzymatical properties if the origins of the enzymes are different. In such a case, it is quite natural that the combination of a particular enzyme with a carrier and a method by which combination a very advantageous immobilized enzyme may be obtained varied depending upon the origins of the enzymes even if the enzymes are given the same enzyme number.

Lactase($\beta$-galactosidase; enzyme number 3. 2. 1. 23) is the enzyme which hydrolyzes lactose into glucose and galactose. It is known that animals, plants, bacteria, fungi and yeast produce lactase. Lactase is a typical example of the enzymes under the name of which there are many kinds of enzymes which are different from each other in their properties except one common property of hydrolyzing lactose into glucose and galactose. For example, of the enzymes originated from bacteria, the lactase derived from *Escherichia coli* (hereinafter "*E. coli*") is the intracellar enzyme of which molecular weight is in a range from $52 \times 10^4$ to $54 \times 10^4$, the optimum pH is 7.3 and Michaelis constant Km (which represents a catalyst-chemical property of an enzyme) is $9.5 \times 10^{-4}$M [substrate; orthonitrophenyl-$\beta$-D-galactopyrano-side(ONPG)], and $7.5 \times 10^{-3}$M (substrate; lactose).

On the other hand, the lactase derived from yeast such as *Sacchromyces fragilis* or *Saccharomyces lactis* is also the intracellar enzyme of which molecular weight is as low as $20 \times 10^4$, less than a half of that of the *E. coli*-originated lactase. The optimum pH of the lactase originated from the yeast is 6.5 to 7.0 and Km values (substrate; ONPG and lactose) are much different from those of the *E. coli*-originated lactase.

Moreover, the lactase originated from a fungus, *Aspergillus niger* is extracellar enzyme of which optimum pH is as low as 3.5 and whose molecular weight is in a range from $10 \times 10^4$ to $11 \times 10^4$. The lactase originated from a similar fungus, *Aspergillus oryzae* has many differences from the *Aspergillus niger*-originated enzymes including the optimum pH being 4.5 to 5.0 (C. D. Boyer: The Enzymes, the third edition, Vol. 7, 617—668).

Thus, it is hardly believed that the lactases whose origins are different from the others have the same chemical structure and properties and are the same compound, protein. They have only one common property in that they all hydrolyze lactose into glucose and galactose or aryl- or alkyl-$\beta$-D-galactoside into an alcohol and galactose.

Accordingly, it may be said that the enzyme-immobilization carrier and the enzyme-immobilization method which are suited to a certain enzyme are not necessarily applicable to all other enzymes. For instance, U.S. Patent 3,767,531 discloses a method of immobilizing the lactase originated from *Aspergillus niger* on phenol-formaldehyde resin which has only phenolic hydroxyl groups and methylol groups but no other functional groups such as amino or substituted amino group or carboxymethyl groups. However, those carriers and method are not necessarily suitable to all kinds of the enzymes.

One major industrial application of lactase is for the hydrolysis of lactose in milk for a person who cannot tolerate lactose in milk, and one other application of lactase is the hydrolysis of lactose in whey. Accordingly, considerable attempts have been made in order to develop microorganism-originated lactases suitable for the said two major industrial applications and the method for immobilizing them [Immobilized Enzyme Technology, edited by H. H. Weetall and S. Suzuki, Plenum Press, New York (1975); Immobilized Enzymes in Food and Microbial Processes, edited by A. C. Olson and C. L. Cooney, Plenum Press, New York (1974)].

In particular, recent developments of techniques on membranes made it possible to separate whey into a high molecular weight protein part and a low molecular weight compound part comprising mainly lactose without difficulties. In view of this, a variety of studies on the hydrolysis of lactose with an immobilized lactase and an application of the product in the field of food industry have been made.

However, almost none of the studies has been put to practical use. The reason for this would be the fact that very few lactase are effective to both milk and acidic whey and that there is a problem of product inhibition by galactose and the like. Among them, the followings would be the main two reasons:

1. Few lactases were stable, highly active to lactose and available at a reasonable price.

2. No available immobilized lactase is stable and highly active to lactose.

As previously discussed, an immobilized lactase is considered more important from the view point of an industrial application of lactase, but no single immobilized method is generally applicable to all kinds of lactases regardless of the origins of the lactase. In order to prepare an excellent immobilized lactase, it is necessary to carefully select the best combination of a lactase, i.e. the kind of the lactase in terms of the origin, a carrier and an immobilizing method. In other words, it is necessary to select the best lactase from the practical point of view, and to decide the most suitable carrier and immobilizing method taking the manner of the industrial application of the resulting product into consideration. This, of course, is applicable to any other immobilized enzymes.

From this standpoint, we have developed new and excellent immobilization-carriers (Japanese Patent Application No. 26913/1978 or published unexamined patent application No. 119084/1979; German Patent Publication (Offenlegungsschrift) No. 2818086)).

It has now been found that the immobilized lactase which has a high activity, and which is so stable that the enzyme is not eluted from the carrier even when a substrate solution of a high salt concentration is used, may be obtained when the lactase originated from Aspergillus oryzae is immobilized on one of the carriers disclosed in the said Japanese published unexamined patent application No. 119084/1979 by covalent attachment with glutaraldehyde, said carrier being macroporous amphoteric phenol-formaldehyde ion-exchange resin having a specific surface area of 1 m²/g or more, the total volume of the macropores having a diameter of 100 to 2,000 Å being 0.1 cc/g or more, the anion-exchange capacity due to amino or substituted amino groups being 1 meq/g or more and the cation-exchange capacity due to carboxymethyl groups being 0.5 meq/g or more. The said carrier is the one disclosed in the said Japanese published unexamined patent application No. 119084/1979.

Accordingly, the present invention provides a water-insoluble, enzymatically-active immobilized lactase having:

(1) an optimum pH with respect to lactose of 4.0 to 5.0, a ratio of activity at pH 4.5 to activity at pH 6.5 according to Figure $2\pm0.1$ (substrate:lactose) when measured at 30°C using a 13.3 w/v solution of lactose and an enzymatically active pH range of pH 2.0 to 8.0.

(2) a Michaelis constant, Km, of $1.5\times10^2\pm10^2$ mole/m³ (pH 4.5, 30°C, substrate; lactose), and

(3) an inhibition constant by galactose, Ki, of $1.5\times10^2\pm10^2$ mole/m³ (pH 4.5, 30°C, substrate; lactose); the immobilised lactase having been obtained by immobilising a lactase obtained from a strain of Aspergillus oryzae, by means of covalent attachment, on a carrier of a macroporous amphoteric phenol-formaldehyde ion-exchange resin having a specific surface area of $10^3$m²/kg or mort, an anion-exchange capacity due to amino or substituted amino groups of 1 eq/kg or more, a cation-exchange capacity due to carboxymethyl groups of 0.5 eq/kg or more and a total pore volume for pores having a diameter of $10^{-8}$ to $2\times10^{-7}$m of $10^{-4}$m³/kg or more; the lactase obtained from a strain of Aspergillus oryzae having:

(1) an optimum pH of from 4.5 to 5.0 (substrate; lactose),

(2) a Michaelis constant, Km, of $10^2\pm50$ mole/m³ (pH 4.5, 30°C substrate; lactose), and

(3) an inhibition constant by galactose, Ki, of $7\pm4$ mole/m³ (pH 4.5, 30°C, substrate; lactose); and the immobilisation having been accomplished by:

(a) adsorbing the lactase obtained from a strain of Aspergillus oryzae on the carrier of a macroporous amphoteric phenol-formaldehyde ion-exchange resin in an aqueous solution of pH 4.0 to 6.5 at from 5°C to 45°C and treating the lactase-adsorbed carrier with an aqueous solution of glutaraldehyde at pH 4.0 to 6.5 and at 5°C to 45°C, or

(b) treating the carrier of a macroporous amphoteric phenol-formaldehyde ion-exchange resin with an aqueous solution of glutaraldehyde at pH 4.0 to 6.5 and at from 5°C to 45°C and reacting the glutaraldehyde bonded to the carrier with the lactase obtained from a strain of Aspergillus oryzae in an aqueous solution having a pH value of 4.0 to 6.5 and at from 5°C to 45°C.

The immobilised enzyme of the invention enables simple and effective use of lactase in a continuous contact reaction of the substrate, lactose.

The immobilized lactase of the present invention has advantages from the standpoint of an industrial utilization of an immobilized enzyme in that, for example, it is active at a wider pH range than the immobilized lactase of which origin is yeast or Aspergillus niger, and retains its activity even at a low temperature lower than 10°C.

A process for producing the said macroporous amphoteric phenol-formaldehyde ion-exchange resin used in the present invention may be any method as far as the produced resin has the above defined properties. The resin specifically disclosed herein for the explanation of the present invention is the one prepared by introducing carboxymethyl groups in a commercially available macroporous anion-exchange resin having a matrix of phenol-formaldehyde condensate, thereby converting the resin to amphoteric ion-exchange resin, as disclosed in the said Japanese published unexamined patent application no. 119084/1979.

The shape of the resin may be granular or bead, and the size may be preferably in a range from 12 mesh (1410 μ) to 60 mesh (250 μ). It is not preferable to use a too large size of resin because the void volume increases in proportion to the size of the resin, and, accordingly, the activity per volume becomes smaller. A too small size of the resin is not preferable either because the pressure drop becomes too large or the separation of the reaction solution from the immobilized enzyme becomes difficult when a too small size of the resin is used. The mesh sizes are according to the JIS standard.

In the accompanying drawings:

Fig. 1 shows the relative activity of the lactase originated from *Aspergillus oryzae* when measured at 30°C using a 13.3 w/v% solution of lactose.

Fig. 2 shows the relative activity of the immobilized lactase of the present invention measured at 30°C using a 13.3 w/v% solution of lactose as the substrate.

The lactase used in the present invention is obtained from a strain belonging to *Aspergillus oryzae* as extracellular enzyme, and has the following characteristics:

1. Optimum pH and pH-dependence

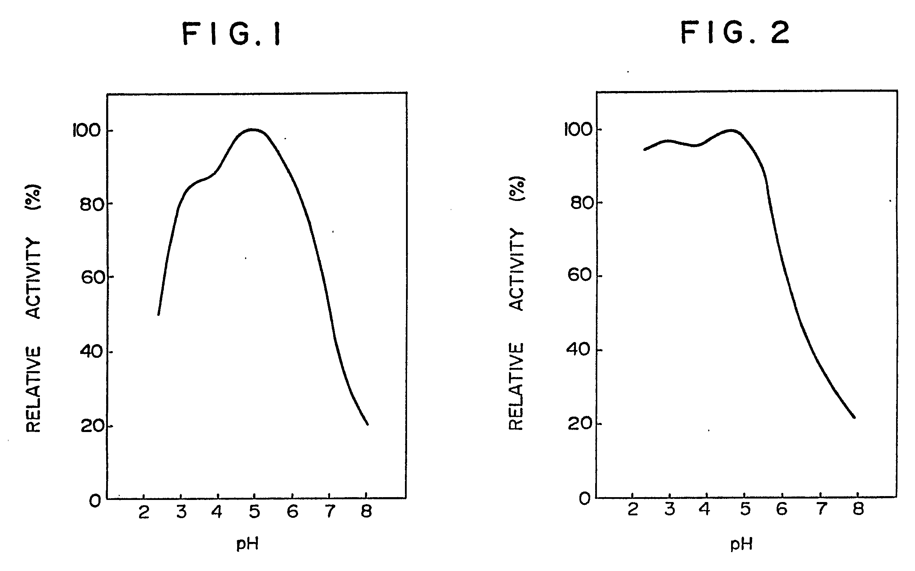

The optimum pH is pH 4.5 to pH 5.0 when the substrate is lactose or ONPG. The pH-dependence of the relative activity for, the substrate, a 13.3 (w/v)% of lactose solution is shown in Fig. 1. The ratio of activity at pH 4.5 to activity at pH 6.5 is according to Figure 1 ±0.05 (substrate:lactose) when measured at 30°C using a 13.3 w/v% solution of lactose.

2. Michaelis constant Km

$Km=10^2\pm50$ mole/m³ (0.10+0.05 mole/l) (pH 4.5, 30°C, substrate; lactose).

3. Inhibition constant by galactose Ki

$Ki=7\pm4$ mole/m³ (0.007±0.004 mole/l) (pH 4.5, 30°C, substrate; lactose). It is believed that the characteristics of the lactase are well defined by the above data, and some other properties are given below:

4. pH-stability

When the lactase was immersed in a buffer solution in the various pH range at 40°C for one hour, the lactase was found to be stable and no decrease of the activity was found in the pH range of 4.0 to 7.5.

5. Temperature-stability

When the lactase was immersed in a buffer solution of pH 4.5 and pH 6.5 at 50°C or lower for 30 minutes, the lactase did not lose its activity at all. The following table shows the residual activity of the enzyme after the enzyme was immersed in at pH 4.5 and pH 6.5 at 55°C or higher for 30 minutes.

| Temperature (°C) | Residual activity (%) |
|---|---|
| 55 | 96—97 |
| 60 | 80 |
| 65 | 20 |

No difference between the results of the experiments at pH 4.5 and pH 6.5 was found.

6. Activation energy, Ea

Arrhenius plots (substrate; 13.3 (w/v)% of lactose solution, pH 4.5) has a curvature slightly and Ea value at 30°C to 40°C is 9±3 kcal/mole.

The above values from 1 to 6 may, of course, include experimental errors permissible in the measurement of enzymatical reactions. As far as the above defined characteristics are satisfied, the process for producing the lactase is not particularly limited. The lactase is usually stored in the form of powder or solution. With respect to the activity, the enzyme of a low activity is meaningless from the gist of the present invention. Accordingly, it is preferable to use the enzyme powder of which enzymatical activity is no less than 15 ILU per mg of the unimmobilized enzyme powder, more preferably no less than 40 ILU/mg.

In the present specification, 1 ILU is defined as the amount of lactase which produces 1 μ mole per minute of glucose at pH 4.5 at 30°C when a 13.3 w/v% of lactose solution is used as the substrate. The quantity of glucose thus prepared is defined by using glycose oxidase-peroxidase-dye system. When the measurement of the activity (unit) is carried out at a different pH or temperature, the pH or the temperature will specifically be given in each case.

The immobilized lactase of the present invention is prepared by one of the following two methods:

One of such methods is carried out in the following procedures:

The carrier is immersed in a solution of lactase in a buffer solution (pH 4.0 to pH 6.5 and at a temperature of 5°C to 45°C), whereby the lactase is adsorbed on the carrier. Then, the carrier adsorbing the lactase is contacted with glutaraldehyde by immersing the carrier in an aqueous solution of glutaraldehyde adjusted to pH 4.0 to pH 6.5 and at a temperature of from 5°C to 45°C, and stirring the mixture to give the immobilized lactase. Thereafter, unreacted glutaraldehyde is washed away. It is believed that glutaraldehyde cross-links between the enzyme and the carrier to immobilize the enzyme on the carrier with a covalent attachment. In fact, the enzyme is not eluted from the carrier even when a high salt concentration of the substrate solution is applied thereto.

The second method is carried out in the reversed orders of the above first method. Namely, the carrier is immersed in an aqueous solution of glutaraldehyde adjusted to pH 4.0 to pH 6.5 and at a temperature of from 5°C to 45°C, thereby reacting glutaraldehyde with the carrier. The carrier thus prepared is then immersed in an aqueous solution of lactase in pH 4.0 to pH 6.5 of a buffer solution and at a temperature of from 5°C to 45°C so that the lactase is reacted the glutaraldehyde bonded to the carrier to give the immobilized enzyme. There is the possibility that a certain amount of enzyme is not reacted with glutaraldehyde to make a covalent attachment, but is simply adsorbed on the carrier. Therefore, the immobilized enzyme thus produced is washed with a high salt concentration of a buffer solution. The immobilized lactases obtained by the first and the second methods as above have the same enzymatical properties with a small difference within the experimental error.

In the first method, it is preferable to wash unadsorbed lactase away after the lactase is subjected to the adsorption operation so that cross-linkings between the unadsorbed, free enzymes are not formed. In the treatment of the carrier adsorbing lactase with glutaraldehyde, the activity is lost when the pH is too low, while the reaction of glutaraldehyde becomes rather inactive in a basic pH range. When the treatment is carried out in a pH range of 4.0 to 6.5, a good result is obtained because the lactase previously adsorbed is not eluted during the glutaraldehyde treatment.

In the second method, in the reaction of glutaraldehyde with the carrier, when the reaction is carried out at a too low pH, it becomes troublesome to convert the pH of the macropores of the carrier reacted with glutaraldehyde to a pH range suitable for the next operation. It is therefore practical to carry out the reaction in the pH range of 4.0 to 6.5. In the next reaction with lactase, a pH range from 4.0 to 6.5 is employed for the same reason as that of the first method.

With respect to the reaction temperature, the adsorption and the reaction with glutaraldehyde are carried out at a temperature from 5°C to 45°C.

In case of the first method, it is preferable to carry out the adsorption at a temperature slightly higher than that of the treatment with glutaraldehyde, for example, carrying out the adsorption at 30°C and the treatment with glutaraldehyde at 20°C.

Suitable concentrations of glutaraldehyde used in the present invention may be 0.1 to 5%. A lower concentration of glutaraldehyde may be used in the first method than in the second method. When the concentration of glutaraldehyde solution is too low, elution of the enzyme occurs and hence the immobilized enzyme of the present invention can hardly be obtained. On the other hand, if the concentration of glutaraldehyde is too high, the enzymatical activity of the immobilized enzyme is decreased. Therefore, a suitable concentration must carefully be selected.

The time necessary for the adsorption, the treatment with glutaraldehyde, the reaction of the carrier with glutaraldehyde and the reaction of enzyme and glutaraldehyde depends upon the temperature of the each process in the immobilization reaction, but generally one to twenty hours is necessary for each operation. In the case where the adsorption and the reaction with glutaraldehyde are carried out at a temperature ranging from 15 to 40°C, 1 to 6 hours is long enough for each operation.

The maximum amount of the lactase to be immobilized is in a range from 250 to 300 mg. per 1 g of dry carrier. Generally, a larger amount of lactase may be immobilized by the first method than the second method.

In carrying out the immobilizing reaction, it is effective to carry out the reaction with stirring at 50 to 250 rpm according to the reaction vessel.

In both the first and second methods, it is very important to wash the immobilized lactase sufficiently with a high salt concentration of a buffer solution and water to remove all the lactase which is insufficiently bonded and hence is likely to be separated. The enzyme which is not separated by such a treatment is the immobilized enzyme of the present invention.

The enzymatic properties of the present immobilized enzymes are given below.

1. Optimum pH and pH-dependency

The optimum pH with respect to lactose is pH 4.0 to pH 5.0. No change in the optimum pH values by the immobilization is observed. However, the activity has a tendency to increase at a lower pH level. The Fig. 2 shows the pH-dependency of the activity of the immobilized enzyme in case that the substrate is a 13.3 w/v% of lactose solution. The ratio of activity at pH 4.5 to activity at pH 6.5 is according to Figure 2 $\pm 0.1$ (substrate:lactose) when measured at 30°C using a 13.3 w/v% solution of lactose.

2. Michaelis constant Km

$Km = 1.5 \times 10^2 \pm 10^2$ mole/m$^3$ ($0.15 \pm 0.1$ mole/l) (pH 4.5, 30°C, substrate; lactose).

3. Inhibition constant by galactose Ki

$Ki = 1.5 \times 10^2 \pm 10^2$ mole/m$^3$ ($0.15 \pm 0.1$ mole/l) (pH 4.5, 30°C, substrate; lactose).

4. pH-stability

When the immobilized enzyme was immersed in a buffer solution at 40°C for one hour, the immobilized enzyme was stable and no decrease of the activity was observed in a pH range from 2.0 to 8.0.

5. Temperature-stability

The immobilized enzyme was immersed at pH 4.5 for 3 hours at a determined temperature. The immobilized enzyme was stable and no decrease of the activity was observed when the temperature was 50°C or lower, but the activity was decreased at a temperature not lower than 55°C.

The immobilized lactase of the present invention has the above stated enzymatical properties and some other practical characteristics thereof are as follows:

1. The activity per unit weight

The activity per 1 g of the dry immobilized lactase is not particularly limited, but the one with a very low activity is worthless in view of the object of the present invention. (Dry immobilized lactase is hereinafter referred to as "IML"). Those having 200 ILU/g-IML or more are useful from the practical point of view at pH 4.5 at 30°C.

As previously stated, the maximum amount of the lactase powder immobilized is 250 to 300 mg/g-carrier. As is clear from the Table 1 below, the activity of the immobilized lactase of the present invention does not largely vary regardless of the amount of the lactase immobilized is in a range from 50 to 200 mg/g-carrier. Activity of the present immobilized lactase depends firstly on that of lactase which is in the powdery or liquid form before being immobilized.

TABLE 1

The activity of the immobilized lactase and amount of the lactase
immobilized at pH 4.5 at 30°C (the activity of the enzyme powder: 72 ILU/mg)

| The amount of the lactase powder immobilized (mg/g-carrier) | 45 | 66 | 89 | 124 | 162 | 224 |
|---|---|---|---|---|---|---|
| The activity of the immobilized lactase (ILU/g-IML) | 785 | 870 | 880 | 840 | 890 | 770 |

2. Good stability and high productivity

The characteristics of the immobilized lactase of the present invention to be particularly noticed are its being highly stabile during storage and at the time when it is subjected to the continuous hydrolysis reaction of lactose, and its high productivity in the hydrolysis reaction of lactose. Even when the substrate contains a high concentration of salt and some other compounds like milk or whey, the immobilized lactase shows a high productivity without causing elution of the enzyme. More specific examples thereof are as follows:

(1) Chemical resistance

The immobilized lactase having 760 ILU/g-IML at pH 4.5 at 30°C was immersed in a dilute solution (concentration: 1/500 of the original solution, this concentration is considered acceptable in food processing industry) of an iodine type disinfectant-detergent, Dyazan® (sold by Asahi Glass K.K.) at 4°C for one month. Thereafter, the activity of the immobilized lactase was measured in the same conditions as above and it was found to possess 765 ILU/g-IML. Thus, no decrease of the activity was observed. The immobilized lactase of the present invention was thus found to be highly resistant to a chemical agent.

(2) Stability in a continuous reaction

Whey powder (made in New Zealand) was dissolved in a buffer solution (pH 4.5) to give a 7 w/v % of whey solution. The undissolved were removed by a centrifuge at 5,000 G to give a nearly transparent whey solution. The whey solution was subjected to the continuous hydrolysis operation at 4°C at a space velocity (SV) of 2.3 hr$^{-1}$ for 135 days with a column packed with 10 ml of the immobilized lactase having 685 ILU/g-IML (pH 4.5, 30°C) of the enzymatical activity to hydrolyze the lactose contained in the whey. The ratio of the hydrolysis of lactose was kept to be 90±3% during the said 135 days, and no decrease of the activity was found. Thus, it is apparent that the immobilized lactase of the present invention is very stable and has a high productivity. The immobilized lactase of the present invention has an advantage from the standpoint of an industrial application. The present invention will be more precisely illustrated by the following examples, but it is not limited thereto.

The conditions of the measurements of the activity of the immobilized lactase of the present invention are explained first as follows:

1. Method for measuring the activity of the immobilized lactase

The immobilized lactase (0.1 to 0.3 ml) is suspended in a 0.05M acetate buffer solution (pH 4.5). To this solution, a solution of lactose in the same kind of buffer solution as above is added to give a 13.3 w/v % lactose solution. The solution was shaken at 30°C for 15 minutes with a shaker (100 rpm, shaking width; 3.5 cm). After the removal of the immobilized lactase by filtration, the content of the glucose in the filtrate is determined using glucose oxidase, peroxidase and dye system. The amount of the enzyme which produces 1 μ mole of glucose in one minute is defined as 1 unit (1 ILU). The pH level and the temperature of the measurement of the activity will be stated in each case in the specification.

2. Measurement of weight of the immobilized lactase and carrier

The immobilized lactase or the carrier is spread out in a 2 mm or less thick layer and dried under reduced pressure (5 mmHg or less) at 50°C for not shorter than 8 hours until a constant weight is reached. Thereafter, the immobilized lactase or the carrier being tested is put in a desicator at a room temperature (18 to 25°C) for 1.5 hours or more and then the weight thereof is measured. This weight is considered to be the weight in the dry state. All of the weights indicated in this specification mean this dry weights.

Example 1
Preparation of immobilized lactase and its composition
15.0 g of the lactase powder originated from *Aspergillus oryzae* (produced by Shinnihon Kagaku Kogyo Co., activity at pH 4.5 at 30°C; 47 ILU, Km at pH 4.5 at 30°C; 0.11 mole/l) was dissolved in 750 ml of 0.05M acetate buffer solution (pH 5.5). In this solution, 100 g of macroporous amphoteric phenol-formaldehyde ion-exchange resin was immersed and the enzymes were immobilized at 20 to 22°C for 6 hours while shaking at 120 RPM. The said resin is the one prepared by introducing carboxymethyl groups into the commercially available Duolite A-7 (trade mark, made by Diamond Shamrock) and has 0.53 cc/g of macropores having a diameter of 100 to 2,000 Å, a specific surface area of about 32 m²/g, a particle size of 250 μ to 1410 μ in diameter, an anion-exchange capacity due to amino and substituted amino groups of 6.08 meq/g and a cation-exchange capacity due to carboxymethyl groups of 2.65 meq/g.

The resin adsorbing the enzymes was then washed with a 0.2M acetate buffer solution (pH 5.5) and deionized water until no enzyme protein was found in the washing liquid. The amount of the enzyme adsorbed on the resin was calculated to be 132.4 mg/g-carrier from the protein content of the washing liquid. Thus obtained resin adsorbing the enzymes was immersed in 750 ml of 1% glutaraldehyde solution adjusted to pH 4.5 and stirred at 120 RPM for 6 hours while maintaining the temperature to 5°C. The resin was sufficiently washed with a 0.2M acetate buffer solution and deionized water, and the amount of the enzyme immobilized on the resin after the treatment with glutaraldehyde was found to be 131.5 mg/g-carrier. The activity of the immobilized lactase prepared as above was 635 ILU/g-IML at pH 4.5 at 30°C. The immobilized lactase (9 ml each) was packed in two columns equipped with a jacket tube. While maintaining the temperature of the columns to 40°C, a 0.3M acetate buffer solution (pH 4.5) was passed through one of the columns at a space velocity of 5.0 hr⁻¹ for 48 hours, and, with the same conditions, a 0.3M phosphate buffer solution (pH 6.65) was passed through the other column. The used buffer solutions have the same electrical conductivity as that of milk or a 7% solution of whey. It was found from the ultraviolet spectrum of the buffer solutions came out from the columns that about 0.5% of the enzyme immobilized was eluted during the first several hours after the buffer solutions were started to pass through. But, no further elution of the enzyme was observed. The activities of the immobilized enzymes at pH 4.5 at 30°C after the buffer solutions were passed through for 48 hours were 645 ILU/g-IML and 640 ILU/g-IML, respectively. Thus, it can be concluded that no substantial decrease of the activity was caused by the operations as above. In other words, it may be said that the immobilized enzyme is so stable that the enzyme is not separated from the carrier even treated with a high salt concentration of solution.

Continuous hydrolysis of lactose in skim milk
A column packed with 30 ml of the immobilized lactase prepared above was placed in a room where the temperature was kept to 4°C and a skim milk solution (sugar content, 5 w/v %, measured by Nelson-Somogyi method) was continuously passed through the column for 180 days at a space velocity of 0.8 hr⁻¹. The pH value of the used skim milk was 6.65, which was not adjusted artificially. The rate of the hydrolysis of lactose in seven days after the skim milk was first started to pass through was 72%, and that of the last day, the 180th day was 71%. Thus, no substantial decrease of the activity was observed. During the above operation, the immobilized enzyme was washed twice a week with a solution of a disinfectant-detergent comprising mainly 2% of iodine and 11% of a non-ionic surfactant, Diazan® sold by Asahi Glass K.K. (concentration used is 1/100 of the original solution) and sterile water. From this fact, it is understood that the activity was not decreased by the disinfectant.

Continuous hydrolysis of lactose in whey
10 ml of the immobilized lactase was packed in a column and the column was placed in a room where the temperature was kept to 4°C. Whey (made in New Zealand) was dissolved in a buffer solution (pH 4.4) to give a 7 w/v % of the whey solution, and the undissolved were removed by a centrifuge at 5,000G. The undissolved material removed from the whey solution was 10% of the whey powder used. This whey solution was continuously passed through the column at a space velocity of 2.25 hr⁻¹ for 200 days to hydrolyze lactose. The rate of the hydrolysis of lactose was 88% for 4 days after the whey solution was started to pass through and 90% at the last day, the 200th day. Thus, no decrease of the activity was observed.

In this experiment, the immobilized lactase was washed once every two weeks with a solution of Diazan® (concentration; 1/500) and sterile water to prevent putrefaction, which is likely to occur when a whey solution is passed through the immobilized enzyme.

Example 2
Preparation of immobilized lactase and its composition
18.2 g in the dry state of the same carrier as used in Example 1 was weighed and immersed in deionized water, thereby swelling the carrier. Thereafter, the carrier was immersed in 150 ml of 3% glutaraldehyde solution (adjusted to pH 4.5) and stirred for 2 hours while keeping the solution temperature to 18±1°C. The carrier was sufficiently washed with deionized water and an acetate buffer solution and then immersed in 150 ml of solution of the same lactase powder (3.0 g) as used in Example 1 in 0.05 M acetate buffer solution (pH 5.0). The mixture was slowly stirred at 25°C for 4 hours to proceed the immobilization reaction. The carrier was washed sufficiently with 0.3 M acetate buffer solution (pH 5.0) and deionized water to remove the enzyme immobilized only by adsorption on the carrier. The amount of the enzyme thus immobilized was found to be 149.4 mg/g-carrier from the calculation based on the amount of the protein in the washing solution, and the activity of the immobilized enzyme was 685 ILU/g-IML at pH 4.5 at 30°C.

The activity of the immobilized enzyme after 0.3M buffer solutions of pH 4.5 and pH 6.65 were passed through the enzyme for 48 hours as Example 1 was 700 ILU/g-IML and 690 ILU/g-IML, respectively. Thus, it may be said that the immobilized enzyme of the present invention is so stable that the activity is not decreased by the separation of the enzyme from the carrier.

Stability of immobilized lactase (chemical-resistance)

The immobilized lactase prepared above was immersed in solutions of disinfectant, Osuban®️ sold by Takeda Chemical Industries, Ltd. which comprises 10 w/v % solution of benzalkonium chloride (concentration; 1/100, 1/500) and allowed to stand for one month at 4°C. The activity of the immobilized enzyme was measured at pH 4.5 at 30°C and found to be 680 ILU/g-IML and 685 ILU/g-IML, respectively. Thus, no decrease of the activity was observed.

Continuous hydrolysis of lactose in low fat milk

The immobilized lactase prepared above (20 ml) was packed in a column, and low fat milk (manufactured by Meiji Nyugyo, Sugar content; 5.25% measured by Nelson-Somogyi method was passed through the column at a space velocity of 0.75 hr$^{-1}$ for 100 days in a room where the temperature was kept to 4°C. The rate of the hydrolysis of lactose was 72±3% throughout the operation period and, thus, no decrease of the activity was observed. In this experiment, the immobilized lactase was sterilized by washing twice a week with an iodine-type disinfectant and sterile water.

Measurement of Km, and Ki

Km value and Ki value of the immobilized lactase prepared above were measured at pH 4.5 of 30°C. The Km value was calculated from Lineweaver-Burk plots to be 0.16 mole/l. The Ki value measured by adding 0.2 M galactose was 0.11 mole/l.

Example 3
Preparation of immobilized lactase and its composition

1.15 g of dry powder of lactase originated from *Aspergillus oryzae* (produced by Shinnihon Kagaku Kogyo Co., activity of the enzyme; 73 ILU/mg at pH 4.5 at 30°C, Km value; 0.11 mole/l) was dissolved in 75 ml of 0.05 M acetate buffer solution (pH 5.5). In this solution, there was immersed 10 g of macroporous amphoteric phenolformaldehyde ion-exchange resin having a particle size of 250 μ to 1,000 μ and a specific surface area of 68 m$^2$/g, the total volume of the macropores having a diameter of 100 to 2,000 Å being 0.56 cc/g, the cation-exchange capacity being 3.62 meq/g, and the anion-exchange capacity due to carboxymethyl groups being 1.85 meq/g (prepared by introducing carboxymethyl groups into a commercially available resin, Duolite A-4 made by Diamond Shamrock). The mixture was stirred for 3 hours at 120 RPM to immobilize lactase on the carrier, while keeping the temperature of the mixture to 35±2°C. Thereafter, the immobilized lactase was washed well with 0.2 M acetate buffer solution (pH 5.5) and deionized water. The amount of the enzyme immobilized was found to be 96 mg/g-carrier. The activity of thus prepared adsorption-immobilized lactase was 1,080 ILU/g-IML at pH 4.5 at 30°C.

This adsorption-immobilized lactase was immersed in 75 ml of a 2.5% glutaraldehyde solution (adjusted to pH 4.3) and stirred at 120 RPM for 4 hours while the temperature of the solution was kept to 10 to 15°C. After this treatment with glutaraldehyde, the amount of the enzyme immobilized was found to be 92 mg/g-carrier from the washing solution. The activity of this immobilized lactase at pH 4.5 at 30°C was 815 ILU/g-IML.

Continuous hydrolysis of lactose

Two columns equipped with a jacket tube were packed with 10 ml each of the immobilized lactase. A 7 w/v % solution of lactose in 0.05 M phosphate buffer solution containing 100 ppm of n-propyl p-hydroxybenzoate was continuously passed through the columns to hydrolyze the lactose. The solution was passed through the first column at pH 6.3 at 40°C at a space velocity of 5.0 hr$^{-1}$, and the second column at pH 4.5 at 40°C at a space velocity of 8.5 hr$^{-1}$. The first column (operated at pH 6.3, and at a space velocity of 5.0 hr$^{-1}$) retained 98±2% of the hydrolysis rate for 100 days and no decrease of the activity was observed at all. However, the immobilized enzyme had to be washed once a week with a solution of the same disinfectant-detergent as used in Example 2, Osuban®️ (concentration; 1/300) because infection by a microorganism was so vigorous. The hydrolysis rate was decreased just before the time when the washing had to be carried out, but recovered to the original value of 98±2% when the immobilized lactase was washed, while the second column (operated at pH 4.5, at a space velocity of 8.5 hr$^{-1}$) retained

98—100% of the hydrolysis rate throughout the 200 days, and no decrease of the activity was observed. Under the operation conditions of the second column, it is sufficient if sterilization and washing were carried out once every two or three weeks.

### Example 4
### Preparation of immobilized lactase and its composition

28 g of the same dry lactase as the one used in Example 3 was dissolved in 1,825 ml of 0.05 M acetate buffer solution (pH 5.2). In this solution, there was immersed macroporous amphoteric phenol-formaldehyde ion-exchange resin (240 g in the dry state) having almost the same particle size, specific surface area and macroporous volume as those of the resin used in Example 1, but having 6.26 meq/g of anion-exchange capacity and 2.86 meq/g of cation-exchange capacity due to carboxymethyl groups. The mixture was stirred for 4 hours at about 150 RPM, while maintaining the temperature of the mixture to 30°C. Thereafter, the immobilized enzyme was washed with a 0.2 M acetate buffer solution (pH 4.5) and deionized water.

By calculating from the amount of the protein contained in the washing solution, the amount of the enzyme adsorbed was found to be 112 mg/g-carrier. The carrier adsorbing lactase was immersed in 1,800 ml of 0.75% glutaraldehyde solution [in 0.05 M acetate buffer solution (pH 4.5)], and stirred for 3 hours at 120 RPM while keeping the temperature of the solution to be 19 to 20°C. Three hours thereafter, the immobilized lactase was sufficiently washed with 0.2 M acetate buffer solution and deionized water. The amount of the enzyme kept immobilized after the above treatment was found to be 111 mg/g. The activity of the immobilized enzyme at pH 4.5 at 30°C was 850 ILU/g-IML.

### pH-dependency of activity

The activity of the immobilized lactase prepared above was measured at 30°C using a 13.3 w/v % of lactose solution. The results expressed by the pH-dependency of the relative activity are consistent with the curve of the Fig. 2 within the experimental error.

### Measurement of Km, Ki

Experiments were conducted twice at pH 4.5 at 30°C to determine the Km and Ki values of the immobilized enzyme prepared in the present example. The average Km and Ki values of the experiments are 0.2 mole/l and 0.15 mole/l, respectively.

### Storage-stability

A part of the immobilized lactase prepared above was slightly dried under reduced pressure. When the water content of the immobilized enzyme became 50% (the immobilized lactase looks dry when the weight of the dry immobilized lactase is equal to that of the water contained therein), the immobilized lactase so dried was divided into two parts. They were put in bottles, which were then sealed in the air. Thereafter, the first bottle was kept at 4°C for two months and the second one at a room temperature (15—28°C) for two months. The activity of the immobilized lactase in the first and second bottles are measured and found to be 830 ILU/g-IML and 870 ILU/g-IML, respectively. Thus, no decrease of the activity was observed at all.

The immobilized lactases were immersed in solutions adjusted to pH 2.6 and pH 3.0 for one month at 4°C, then the activities of the enzymes so immersed were measured at pH 4.5 at 30°C and found to be 845 ILU/g-IML in both cases. Thus, the decrease of the activity was within the experimental error.

### Continuous hydrolysis of lactose in skim milk

The immobilized lactase (11.3 ml) prepared above was packed in a column and skim milk (the concentration of lactose thereof was adjusted to 5.0 w/v %) was passed through the column at a space velocity of 1.0 hr$^{-1}$ in a room where the temperature was kept to 4°C. During the 100 days of the continuous operation, the hydrolysis rate was 72±2% and no decrease of the activity was observed at all. During this experiment, the immobilized lactase was washed twice a week with a solution of an iodine-type disinfectant (dilution rate 1/100).

### Example 5
### Preparation of immobilized lactase and its composition

30 g of lactase originated from *Aspergillus oryzae* (80 ILU at pH 4.5, 30°C), was dissolved in 1750 ml of acetate buffer solution (pH 5.5). In this solution, there was immersed 230 g of the same resin as the one used in Example 4 and stirred for 4 hours at 120 RPM, while maintaining the temperature of the solution to 30°C, thereby adsorbing the enzyme. The amount of the enzyme adsorbed was found to be 121.4 mg/g-carrier from the amount of the protein contained in the washing solution. Thereafter, the carrier having the enzyme immobilized by adsorption was immersed in 1,750 ml of 0.5% glutaraldehyde solution (adjusted to pH 4.5 with 0.05 M acetate buffer solution), and stirred for 5 hours at 120 RPM while keeping the temperature of the solution to 20°C. The amount of the lactase immobilized was found to be 121.1 mg/g-carrier from the washing solution. The activity of thus prepared immobilized lactase was 1,000 ILU/g-IML at pH 4.5 at 30°C.

### Continuous hydrolysis of lactose in skim milk

20 ml of the immobilized lactase prepared in the present example was packed in a column and a skim milk solution (the sugar content was adjusted to 5.0 w/v %) was passed through the column at a space velocity of 1.2 hr$^{-1}$ in a room where the temperature was kept to 4°C. The reaction was continued for 135 days, during which the rate of the hydrolysis of lactose was in a range from 70 to 75%, thus, no decrease of the activity was observed. During this operation, the immobilized lactase was sterilized and washed twice a week.

Continuous hydrolysis of lactose in whey

The immobilized lactase (9 ml each) was packed in two columns. A solution (7 w/v %) of whey powder (made in New Zealand) containing 150 ppm of n-propyl p-hydroxybenzoate was centrifuged at 5,000 G to remove the undissolved. The supernatant (adjusted to pH 4.5) was passed through one of the columns at a space velocity of 3.5 hr$^{-1}$ in a room kept at 4°C and the other column at 40°C with a space velocity of 16 hr$^{-1}$. In the latter case, the solution of the substrate and reaction solution came out from the column were cooled to 2°C to prevent putrefaction. In both cases, the continuous reaction was carried out for 135 days. The former column retained 88±2% of the rate of the hydrolysis of lactose during the period of the operation. The latter column retained 97 to 100% of the rate of the hydrolysis of lactose throughout the operation period, and no decrease of the activity was observed. With respect to the former column, when the immobilized enzyme in the column was sterilized once every two weeks, an infection by microorganism was avoided. But, in case of the latter, an infection could not be avoided even when the immobilized enzyme was sterilized once a week, and the rate of the hydrolysis was decreased. However, the hydrolysis rate was recovered when the enzyme was sterilized.

Comparative Example 1
Immobilization of lactase originated from yeast and activity of the product

Dry powder of lactase originated from a yeast, *Saccharomyces (Kluyveromyces) lactis* was adsorbed on the same carrier as the one used in Example 1 at pH 6.65, and treated with a 1.0% glutaraldehyde solution (pH 6.65). The amount of the enzyme powder immobilized was 96 mg/g-carrier and the rate of the enzyme immobilization was 64%. However, a big practical problem thereof is that the activity of the immobilized enzyme was 1.4 ILU/g-IML when measured at pH 6.65 at 30°C using a 13.3% (w/v) lactose solution as the substrate, which means that the enzyme has no activity in a practical sense.

Moreover, when this immobilized lactase was washed with an acetate buffer solution (pH 5.5), the activity of the enzyme was completely lost. From this, it is understood that the immobilized lactase prepared by immobilizing the lactase originated from *Saccharomyces lactis* in accordance with the immobilizing method of the present invention has no practical value.

Comparative Example 2
Immobilization on a carrier having neither amino nor carboxymethyl group

By using the same lactase powder as used in Example 1, the immobilization was carried out in exactly the same manner except that a commercially available resin, Duolite ES-762 (manufactured by Diamond Shamrock; it has no ion-exchange group other than phenolic hydroxyl or methylol groups) was used instead and the experiment was carried out in 1/10 of scale of the Example 1. The amount of the enzyme immobilized was 53 mg/g-carrier and the activity at pH 4.5 at 30°C was 285 ILU/g-IML, less than a half of the value of the Example 1. A certain amount of the immobilized lactase of Example 1 and the one prepared in this Comparative Example 2 were immersed in a solution of a disinfectant-detergent, Diazan® (dilution rate; 1/100) at a room temperature (15—22°C). The activity of the latter immobilized lactase decreased to 90% in 24 hours, and to 82% in 5 days, but that of the former was not decreased during the same period.

Comparative Example 3
Immobilization of lactase originated from Aspergillus niger

The industrial grade of lactase originated from *Aspergillus niger* was adsorbed on the carrier of the present invention, and treated with a 2.0% glutaraldehyde solution (pH 4.5). The activity of this immobilized lactase was less than 1 ILU/g-IML at pH 6.65, which is the pH value of milk, at 30°C and thus this immobilized lactase has no practical value for the purpose of the hydrolysis of lactose in milk.

**Claims**

1. A water-insoluble, enzymatically-active immobilized lactase having:

(1) an optimum pH with respect to lactose of 4.0 to 5.0, a ratio of activity at pH 4.5 to activity at pH 6.5 according to Figure 2±0.1 (substrate:lactose) when measured at 30°C using a 13.3 w/v % solution of lactose and an enzymatically active pH range of pH 2.0 to 8.0,
(2) a Michaelis constant, Km, of $1.5 \times 10^2 \pm 10^2$ mole/m$^3$ (pH 4.5, 30°C, substrate:lactose), and
(3) an inhibition constant by galactose, Ki, of $1.5 \times 10^2 \pm 10^2$ mole/m$^3$ (pH 4.5, 30°C, substrate:lactose);

the immobilized lactase having been obtained by immobilizing a lactase obtained from a strain of *Aspergillus oryzae*, by means of covalent attachment, on a carrier of a macroporous amphoteric phenol-formaldehyde ion-exchange resin having a specific surface area of 10$^3$m$^2$/kg or more, an anion-exchange capacity due to amino or substituted amino groups of 1 eq/kg or more, a cation-exchange capacity due to carboxymethyl groups of 0.5 eq/kg or more and a total pore volume for pores having a diameter of 10$^{-8}$ to $2 \times 10^{-7}$m of 10$^{-4}$m$^3$/kg or more; the lactase obtained from a strain of *Aspergillus oryzae* having:

(1) an optimum pH of from 4.5 to 5.0 (substrate:lactose),

(2) a Michaelis constant, Km, of $10^2 \pm 50$ mole/$m^3$ (pH 4.5, 30°C, substrate:lactose), and

(3) an inhibition constant by galactose, Ki, of $7 \pm 4$ mole/$m^3$ (pH 4.5, 30°C, substrate:lactose); and

the immobilization having been accomplished by:

(a) adsorbing the lactase obtained from a strain of *Aspergillus oryzae* on the carrier of a macroporous amphoteric phenol-formaldehyde ion-exchange resin in an aqueous solution of pH 4.0 to 6.5 at from 5°C to 45°C and treating the lactase-adsorbed carrier with an aqueous solution of glutaraldehyde at pH 4.0 to 6.5 and at 5°C to 45°C., or

(b) treating the carrier of a macroporous amphoteric phenol-formaldehyde ion-exchange resin with an aqueous solution of glutaraldehyde at pH 4.0 to 6.5 and at from 5°C to 45°C and reacting the glutaraldehyde bonded to the carrier with the lactase obtained from a strain of *Aspergillus oryzae* in an aqueous solution having a pH value of 4.0 to 6.5 and at from 5°C to 45°C.

2. An immobilized lactase according to claim 1, wherein the activity per gram of the dry immobilized lactase is 200 ILU or more at pH 4.5 and 30°C.

3. An immobilized lactase according to claim 1 or 2 wherein, in immobilization method (a), the adsorption of the lactase on the carrier is carried out at a slightly higher temperature than the temperature of the glutaraldehyde treatment.

4. A process for hydrolysing lactose, which process comprises contacting lactose with an immobilised lactase as claimed in any one of claims 1 to 3.

## Patentansprüche

1. Wasserunlösliche, enzymatisch wirksame, immobilisierte Lactase, mit

(1) einem optimalen pH-Bereich gegenüber Lactose von 4,5 bis 5,0, einem Verhältnis der Wirksamkeit bei pH 4,5 zur Wirksamkeit bei pH 6,5 entspechend Fig. 2 von ±0,1 (Substrat:Lactose), wenn die Messung bei 30°C unter Verwendung einer 13,3 w/v %-igen Lösung von Lactose erfolgt und einem enzymatisch wirksamen pH-Bereich von pH 2,0 bis 8,0,

(2) einer Michaelis-Konstanten Km von $1,5 \times 10^2 \pm 10^2$ Mole/$m^3$ (pH 4,5, 30°C, Substrat:Lactose), und

(3) einer Inhibierungskonstante durch Galactose Ki von $1,5 \times 10^2 \pm 10^2$ Mole/$m^3$ (pH 4,5, 30°C, Substrat:Lactose);

wobei die immobilisierte Lactase erhalten ist durch Immobilisierung einer vom Stamm Aspergillus Oryzae erhaltenen Lactase über eine kovalente Bindung an einem Trägermaterial aus einem makroporösen, amphoteren Phenol-Formaldehyd-Ionenaustauscherharz mit

einer spezifischen Oberfläche von $10^3 m^2$/kg oder mehr, einer Anionenaustauscherkapazität aufgrund der Aminogruppen und/oder substituierten Aminogruppen von 1 eq/kg oder mehr, einer Kationenaustauscherkapazität aufgrund der Carboxymethylgruppen von 0,5 eq/kg oder mehr und einem Gesamtporenvolumen für Poren mit einem Durchmesser von $10^{-8}$ bis $2 \times 10^{-7}$ m von $10^{-4} m^3$/kg oder mehr;

wobei die vom Stamm Aspergillus Oryzae erhaltene Lactase:

(1) einen optimalen pH-Bereich von 4,5 bis 5,0 (Substrat:Lactose),

(2) eine Michaelis-Konstante Km von $10^2 \pm 50$ Mole/$m^3$ (pH 4,5, 30°C, Substrat:Lactose), und

(3) eine Inhibierungskonstante durch Galactose Ki von $7 \pm 4$ Mole/$m^3$ (pH 4,5, 30°C, Substrat:Lactose) aufweist; und

die Immobilisierung erreicht wurde durch:

(a) Adsorption der vom Stamm Aspergillus Oryzae erhaltenen Lactase an dem Trägermaterial aus einem makroporösen, amphoteren Phenol-formaldehyd-Ionenaustauscherharz in einer wäßrigen Lösung von pH 4,0 bis 6,5 bei 5°C bis 45°C und Behandlung des Trägermaterials, an dem die Lactase adsorbiert ist, mit einer wäßrigen Glutaraldehydlösung bei pH 4,0 bis 6,5 und bei 5°C bis 45°C, oder

(b) Behandeln des Trägermaterials aus einem makroporösen amphoteren Phenol-formaldehyd-Ionenaustauscherharz mit einer wäßrigen Glutaraldehydlösung bei pH 4,0 bis 6,5 und bei 5°C bis 45°C und Reagierenlassen des an das Trägermaterial gebundenen Glutaraldehyds mit der vom Stamm Aspergillus Oryzae erhaltenen Lactase in einer wäßrigen Lösung mit einem pH-Wert von 4,0 bis 6,5 bei 5°C bis 45°C.

2. Immobilisierte Lactase nach Anspruch 1, bei der die Wirksamkeit pro Gramm der trockenen, immobilisierten Lactase bei pH 4,5 und 30°C 200 ILU oder mehr ist.

3. Immobilisierte Lactase nach Anspruch 1 oder 2, bei der bei der Immobilisierungsart (a) die Adsorption der Lactase an dem Trägermaterial bei einer leicht höheren Temperatur als die Temperatur der Glutaraldehydbehandlung ausgeführt wird.

4. Verfahren für die Hydrolyse von Lactose, bei dem man Lactose mit einer immobilisierten Lactase gemäß einem der Ansprüche 1 bis 3 behandelt.

## Revendications

1. Lactase immobilisée, insoluble dans l'eau et active du point de vue enzymatique, ayant:

(1) un pH optimum vis-à-vis du lactose de 4,0 à 5,0, un rapport de l'activité à pH 4,5 à l'activité à pH 6,5 suivant la figure 2, de ±0,1 (substrat:lactose) telle que mesurée à 30°C en utilisant une solution de lactose à 13,3% en poids/volume et une gamme de pH d'activité enzymatique allant de 2,0 à 3,0,

(2) une constante de Michaelis, Km, de $1,5\times10^2\pm10^2$ moles/m$^3$ (pH 4,5, 30°C, substrat:lactose), et

(3) une constante d'inhibition par le galactose, Ki, de $1,5\times10^2\pm10^2$ moles/m$^3$ (pH 4,5, 30°C, substrat:lactose);

la lactase immobilisée ayant été obtenue en immobilisant une lactase obtenue d'une souche d'*Aspergillus oryzae*, au moyen d'une liaison covalente sur un support en une résine macroporeuse amphotère d'échange d'ion en phénol-formaldéhyde, ayant une surface spécifique de $10^3$m$^2$/kg ou supérieure à cette valeur, et une capacité d'échange d'anions due aux groupes ou aux groupes amino substitués de 1 eq/kg ou supérieure à cette valeur, une capacité d'échange de cations due aux groupes carboxyméthyle de 0,5 eq/kg ou supérieure à cette valeur, et un volume total de pore pour les pores ayant un diamètre de $10^{-8}$ à $2\times10^{-7}$ m de $10^{-4}$m$^3$/kg ou supéreiure à cette valeur;

la lactase obtenue d'une souche d'*Aspergillus Oryzae* ayant:

(1) un pH optimum de 4,5 à 5,0 (substrat:lactose),

(2) une constante de Michaelis, Km, de $10^2\pm50$ moles/m$^3$ (pH 4,5, 30°C, substrat:lactose), et

(3) une constante d'inhibition par le galactose, Ki, de $7\pm4$ moles/m$^3$ (pH 4,5, 30°C, substrat:lactose); et l'immobilisation étant effectuée:

(a) en adsorbant entre 5°C et 45°C la lactase obtenue d'une souche d'*Aspergillus oryzae* sur le support d'une résine macroporeuse amphotère d'échange d'ion en phénol-formaldéhyde, en solution aqueuse d'un pH de 4,0 à 6,5 et en traitant entre 5 et 45°C le support ayant adsorbé de la lactase par une solution aqueuse de glutaraldéhyde d'un pH de 4,0 et 6,5, ou

(b) en traitant entre 5°C et 45°C le support en une résine macroporeuse amphotère d'échange d'ion en phénol-formaldéhyde par une solution aqueuse de glutaraldéhyde, ayant un pH de 4,0 à 6,5, et en faisant réagir entre 5°C et 45°C le glutaraldéhyde lié au support sur la lactase obtenue d'une souche d'*Aspergillus oryzae* en solution aqueuse ayant un pH de 4,0 à 6,5.

2. Lactase immobilisée suivant la revendication 1, dans laquelle l'activité par gramme de la lactase sèche immobilisée est de 200 ILU ou supérieure à cette valeur, à un pH de 4,5 et à 30°C.

3. Lactase immobilisée suivant la revendication 1 ou 2, dans laquelle, dans le procédé d'immobilisation (a), l'adsorption de la lactase sur le support est effectuée à une température légèrement supérieure à la température du traitement par le glutaraldéhyde.

4. Procédé pour hydrolyser du lactose, ce procédé consistant à mettre du lactose en contact avec une lactase immobilisée, telle que revendiquée suivant l'une quelconque des revendications 1 à 3.

# F I G . 2

# F I G . 1